# FASCICULE DE BREVET EUROPEEN

(11) **EP 1 398 055 B1**
(45) Date de publication et mention de la délivrance du brevet: **23.08.2006**
(21) Numéro de dépôt: 03292261.9
(22) Date de dépôt: 12.09.2003
(51) Int. Cl.: A61N 1/362, A61N 1/39

(54) **Dispositif médical implantable actif du type défibrillateur, cardioverteur et/ou stimulateur antitachycardique, à fréquence maximale élevée de stimulation antibradycardique**
Implantierbares medizinisches aktives Gerät, wie Defibrillator, Kardioverter und/oder Herzschrittmacher zur Antitachykardie, mit Stimulation zur Antibradykardie mittels einer hohen maximalen Frequenz
Implantable active medical device, like a defibrillator, cardioverter and/or antitachycardia pacing, with anti-bradycardia pacing using a high maximal frequency

(30) Priorité: 16.09.2002 FR 0211430
(43) Date de publication de la demande: 17.03.2004
(73) Titulaire: ELA MEDICAL, 92541 Montrouge (FR)
(72) Inventeur: Henry, Christine, 75014 Paris (FR); Graindorge, Laurence, 92290 Chatenay Malabry (FR)
(74) Mandataire: Dupuis-Latour, Dominique

(56) Documents cités:
- EP-A- 0 550 342
- EP-A- 0 626 182
- EP-A- 0 838 235

## Description

La présente invention concerne les dispositifs médicaux implantables actifs (au sens de la directive 90/385/CEE du 20 juin 1990 du Conseil des communautés européennes), et plus particulièrement la famille des appareils chargés de délivrer au coeur des impulsions électriques de haute énergie (c'est-à-dire dépassant notablement l'énergie fournie pour la simple stimulation) en vue de mettre fin à une tachyarythmie. Ces modes de thérapie incluent également un mode de stimulation programmée à haute fréquence ou "ATP" (*AntiTachycardia Pacing*).

Ces dispositifs sont communément appelés "défibrillateurs implantables" ou "appareils de cardioversion" (étant entendu que l'invention couvre aussi bien les défibrillateurs/cardioverteurs/stimulateurs implantables que les défibrillateurs/stimulateurs implantables).

La décision d'appliquer une thérapie antitachycardique, et le choix de cette thérapie (choc ou stimulation ATP) est opérée par un algorithme de détection et de classification des différentes tachyarythmies en fonction de plusieurs critères, principalement en fonction de la fréquence ventriculaire, mais également en fonction de la stabilité des intervalles ventriculaires, de la stabilité de conduction auriculo-ventriculaire, du mode de démarrage des tachycardies, etc. (voir notamment les EP-A-0 626 182 et

EP-A-0 838 235 au nom de ELA Médical).

La fréquence ventriculaire est un premier critère qui permet en particulier de distinguer trois situations :
- fréquence inférieure à un seuil donné, dit "fréquence de détection de TV (tachycardie ventriculaire)", par exemple de l'ordre de 140 cpm : l'algorithme considère que ce rythme, lent, n'est pas pathologique, ce qui ne justifie jamais l'application d'une thérapie ;
- fréquence comprise entre la fréquence de détection de TV, typiquement 140 cpm, et une autre fréquence, supérieure, dite "fréquence de détection de FV (fibrillation ventriculaire)", typiquement 200 cpm : l'algorithme considère qu'il y a "suspicion de TV" et opère une analyse plus approfondie, mettant en oeuvre d'autres critères que la fréquence ventriculaire, pour déterminer plus précisément le type de trouble et décider s'il y a lieu ou non d'appliquer une thérapie, et quel type de thérapie (choc ou stimulation ATP) ;
- fréquence supérieure à la fréquence de détection de FV, typiquement 200 cpm : l'algorithme considère que l'application d'une thérapie est en tout état de cause nécessaire, et sans délai.

Les dispositifs précités comprennent, outre les moyens de thérapie antitachycardiques que l'on vient d'évoquer, des moyens de stimulation antibradycardiques qui permettent, comme avec un stimulateur implantable classique, de délivrer si nécessaire des impulsions de stimulation au ventricule (et éventuellement à l'oreillette) en l'absence de dépolarisation spontanée de la cavité.

Cette stimulation est opérée à une fréquence variable, dépendant de l'activité du patient. Les stimulateurs peuvent être dotés à cet effet de capteurs physiologiques (par exemple capteur de ventilation-minute) ou de capteurs d'activité (par exemple capteur d'accélération) permettant d'évaluer l'activité instantanée du patient et piloter en conséquence la fréquence de stimulation.

Ainsi, lorsque le patient est à l'effort, le capteur augmente la fréquence de stimulation afin de lui permettre de fournir un effort plus important.

Bien entendu, cette fréquence variable de stimulation est plafonnée, à une valeur dite "fréquence maximale de stimulation", valeur qui est préprogrammée par le praticien au moment de l'implantation ou du suivi.

Une situation particulière se présente lorsque la fréquence maximale de stimulation peut être réglée à une valeur supérieure à la fréquence de seuil de détection des TV. Dans ce cas, pour des valeurs élevées de la fréquence de stimulation, le dispositif considèrera que ce rythme élevé est une tachycardie sinusale (TS), car le rythme est en 1:1, stable et sans accélération.

Mais il peut arriver qu'une TV débute alors même que le patient est dans cette situation, c'est-à-dire une situation d'effort où il est stimulé par le dispositif à une fréquence relativement élevée.

La fréquence de la TV et celle de la stimulation peuvent être voisines, de sorte que la stimulation atriale peut masquer les complexes R et conduire à un retard dans la détection de la TV ou à une sous-détection de la TV, entraînant une absence de thérapie, ou un retard dans l'application de cette thérapie - retard pouvant parfois atteindre plusieurs minutes.

L'un des buts de l'invention est d'éviter une telle situation, en empêchant qu'une stimulation (auriculaire ou ventriculaire) ne masque un événement ventriculaire spontané en cas de suspicion de TV, afin de pouvoir procéder sans retard au diagnostic de TV et assurer dans les plus brefs délais la détection et le traitement de cette TV.

Certes, pour s'affranchir de cette difficulté, les praticiens programment souvent la fréquence maximale de stimulation à une valeur inférieure à la fréquence de détection de TV. Mais ceci a pour inconvénient de limiter la fréquence maximale à laquelle le patient peut être stimulé. Ceci conduit par exemple à limiter la fréquence maximale de stimulation à 140 cpm, alors qu'il serait souhaitable, notamment pour des patients jeunes, d'augmenter cette fréquence, par exemple jusqu'à 180 cpm, afin de leur permettre de leur fournir un effort plus important.

L'invention a également pour but d'éviter cette contrainte, en offrant aux patients qui le peuvent une stimulation antibradycardique pouvant atteindre un rythme élevé, ceci sans compromis sur le diagnostic et le traitement des éventuelles TV.

Le dispositif médical de l'invention est un défibrillateur ou cardioverteur de type en lui-même connu tel que décrit par exemple par le EP-A-0 838 235 précité, c'est-à-dire comprenant des moyens de recueil de l'activité cardiaque ventriculaire et auriculaire, des moyens de thérapie antitachycardique, aptes à appliquer un choc de défibrillation, un choc de cardioversion et/ou une stimulation antitachycardique, et des moyens de stimulation antibradycardique, aptes à délivrer des impulsions de stimulation ventriculaire et/ou auriculaire à une fréquence de stimulation déterminée par le dispositif selon l'activité du patient. Cette fréquence de stimulation est au plus égale à une fréquence maximale de stimulation préprogrammée, et la cadence des impulsions délivrées est définie d'après un intervalle d'échappement calculé incluant un délai atrio-ventriculaire et un délai ventriculo-auriculaire. Il est en outre prévu des premiers moyens d'analyse, mis en oeuvre lorsque les moyens de recueil indiquent une fréquence d'activité cardiaque ventriculaire supérieure à une fréquence de seuil d'analyse donnée, et aptes à reconnaître et discriminer la présence d'une tachycardie ventriculaire, ou d'une fibrillation ventriculaire, et à commander en conséquence les moyens de thérapie antitachycardique.

Selon l'invention, ladite fréquence maximale de stimulation préprogrammée est supérieure à ladite fréquence de seuil d'analyse, et il est prévu en outre des seconds moyens d'analyse du rythme cardiaque, aptes à détecter dans le rythme cardiaque une succession particulière d'événements susceptibles de révéler la présence ou l'apparition d'une tachycardie ventriculaire, et aptes à prolonger la durée dudit délai ventriculo-auriculaire (DVA) au moins jusqu'à la fin dudit intervalle d'échappement calculé et au moins jusqu'à la fin d'un intervalle maximal préprogrammé de détection de tachycardies ventriculaires (correspondant à ladite fréquence de seuil d'analyse (seuil de détection des TV ou TDI)), augmenté d'un délai de tolérance ? de quelque millisecondes (soit : DVA = Max {IE ; TDI+ ?}).

Les seconds moyens d'analyse du rythme cardiaque sont en particulier des moyens aptes à détecter :
- la survenue d'une extrasystole ventriculaire, et/ou
- la survenue d'un événement ventriculaire présentant un intervalle de couplage inférieur ou égal audit intervalle maximal préprogrammé de détection de tachycardies ventriculaires, en présence d'une accélération avérée du rythme d'origine ventriculaire, et/ou
- la survenue d'un événement ventriculaire présentant un intervalle de couplage inférieur ou égal audit intervalle maximal préprogrammé de détection de tachycardies ventriculaires, en présence d'une extrasystole ventriculaire précédant cet événement, la durée dudit intervalle de couplage étant égale, à un facteur de tolérance donné près, à la durée séparant l'extrasystole de l'événement ventriculaire, et/ou
- la présence d'une rythme confirmé de tachycardie ventriculaire.

Plus précisément, les seconds moyens d'analyse sont aptes à prolonger la durée dudit délai ventriculo-auriculaire jusqu'au plus tardif des deux instants correspondant, d'une part, à la fin de l'intervalle d'échappement calculé et, d'autre part, à la fin de l'intervalle maximal préprogrammé de détection de tachycardies ventriculaires, augmenté d'un facteur de sécurité prédéterminé, notamment d'une durée fixe préprogrammée.

Lorsque les moyens de stimulation antibradycardique délivrent des impulsions de stimulation auriculaires synchrones d'une extrasystole ventriculaire, les seconds moyens d'analyse sont avantageusement aptes à prolonger la durée de l'intervalle d'échappement auriculaire déclenché sur une stimulation auriculaire synchrone, cette prolongation étant opérée jusqu'au plus tardif des deux instants correspondant, d'une part, à la fin de l'intervalle d'échappement auriculaire calculé et, d'autre part, à la fin de l'intervalle maximal préprogrammé de détection de tachycardies ventriculaires, augmenté d'un facteur de sécurité prédéterminé.

Lorsque les moyens de stimulation antibradycardique opèrent en mode VVI, les seconds moyens d'analyse sont avantageusement aptes à prolonger la durée de l'intervalle d'échappement ventriculaire déclenché sur un événement ventriculaire présentant un intervalle de couplage inférieur à l'intervalle maximal préprogrammé de détection de tachycardies ventriculaires, et en présence d'une accélération du rythme d'origine ventriculaire, cette prolongation étant opérée jusqu'au plus tardif des deux instants correspondant, d'une part, à la fin de l'intervalle d'échappement ventriculaire calculé et, d'autre part, à la fin de l'intervalle maximal préprogrammé de détection de tachycardies ventriculaires, augmenté d'un facteur de sécurité prédéterminé.
?

Un mode de réalisation détaillé de la présente invention va maintenant être exposé.

On va tout d'abord donner un certain nombre de définitions utilisées dans la suite de la description.
*Détection P* : recueil d'une activité spontanée ayant son origine dans l'oreillette.
*Détection R* : recueil d'une activité spontanée ayant son origine dans le ventricule.
*Stimulation A* : stimulation délivrée à l'oreillette.
*Stimulation V:* stimulation délivrée au ventricule.
*Événement auriculaire* : détection P ou bien stimulation A ;
*Événement ventriculaire* : détection R ou bien stimulation V ;
*Cycle cardiaque* : intervalle de temps séparant deux événements de même nature dans la même cavité, par exemple séparant deux détections P, ou deux stimulations A, ou deux détections R, ou deux stimulations V.
*Intervalle d'échappement (IE)* : intervalle de temps, compté après une détection ou une stimulation dans une cavité donnée, à l'issue duquel une stimulation est délivrée à cette cavité si aucun événement spontané n'a été détecté dans cette même cavité. L'intervalle d'échappement est constitué par la succession du délai atrio-ventriculaire (DAV) et du délai ventriculo-auriculaire (DVA) : IE = DAV + DVA.
*Extrasystole ventriculaire (ESV)* : on définit trois types d'ESV :
· ESV du premier type (ESV1) : extrasystole ventriculaire simple, c'est-à-dire détection R sans détection P associée, dans un intervalle de temps donné, typiquement [31 ; 300 ms] ;
· ESV du deuxième type (ESV2) : détection R précédée d'une détection P ou d'une stimulation V, avec un intervalle de couplage (intervalle R-R ou intervalle V-R) inférieur ou égal à une valeur paramétrable, par exemple 75 %, du PP moyen - c'est-à-dire un intervalle de couplage inférieur ou égal audit intervalle maximal préprogrammé de détection de tachycardies ventriculaires, en présence d'une accélération avérée du rythme d'origine ventriculaire ;
· ESV du troisième type (ESV3) : événement ventriculaire présentant un intervalle de couplage inférieur ou égal audit intervalle maximal préprogrammé de détection de tachycardies ventriculaires, en présence d'une ESV précédant cet événement, la durée de cet intervalle de couplage étant égale, à un facteur de tolérance donné près, à la durée séparant l'extrasystole de l'événement ventriculaire.

Pour de plus amples détails sur les extrasystoles, on pourra se référer au EP-A-0 550 342 (Ela Médical), qui décrit un algorithme de détection et de traitement des ESV.

La présente invention peut être mise en oeuvre à partir de l'algorithme déjà connu et décrit dans les EP-A-0 626 182 et EP-A-0 838 235 précités, et qui est utilisé par les modèles de défibrillateurs DEFENDER et ALTO d'ELA Médical pour opérer la détection et la classification des différentes tachyarythmies en fonction de divers critères.

Cet algorithme permet en particulier de détecter et confirmer la survenue de TV par une analyse du rythme cardiaque, cette analyse étant mise en oeuvre dès que la fréquence ventriculaire du rythme recueilli dépasse une fréquence programmée dite "fréquence de détection de TV". Il est en particulier possible de discriminer, entre divers troubles, ceux qui autorisent l'application d'une thérapie antitachycardique (TV lentes ou rapides avérées, FV), et, d'autre part, ceux d'origine non ventriculaire, pour lesquels toute thérapie de ce type doit être inhibée : tachycardies supra-ventriculaires ("TSV"), tachycardies sinusales ("TS") et troubles analogues (on pourra se référer aux brevets précités pour de plus amples détails).

Par ailleurs, le dispositif comprend des moyens de stimulation antibradycardiques, c'est-à-dire qu'il permet un fonctionnement traditionnel en stimulateur cardiaque,en autorisant une fréquence de stimulation pouvant atteindre une "fréquence maximale de stimulation".

L'invention vise le cas particulier où cette fréquence maximale de stimulation peut être ajustée à une valeur (par exemple 180 cpm) supérieure à la fréquence minimale de détection des TV (par exemple 140 cpm).

L'invention vise à éviter qu'une stimulation, auriculaire ou ventriculaire, ne masque un événement ventriculaire spontané en cas de suspicion de TV, de manière à assurer une détection et un traitement rapide de cette TV.

Pour ce faire, lorsque le dispositif fonctionne en mode double chambre, il prolonge si nécessaire le délai auriculo-ventriculaire ("délai VA") de manière à ce que celui-ci se termine à un instant correspondant au plus tardif de :
- la fin de l'intervalle d'échappement calculé : IE calculé peut être l'intervalle d'échappement de base, ou bien il peut être donné par une fonction d'asservissement du stimulateur, ou bien encore par des fonctions de lissage, ou de prévention, ou une combinaison de ces fonctions, ceci de manière en elle-même connue ;
- la fin de l'intervalle maximal programmé de détection de TV (en général, l'intervalle de détection de TV lente, correspondant à la fréquence minimale de détection des TV), augmenté d'une marge de sécurité (par exemple une valeur programmable, typiquement 31 ms), pour assurer la détection d'une TV exactement à la fréquence de détection des TV).

Cette prolongation éventuelle du délai VA est opérée sur un cycle seulement (au cycle suivant, l'algorithme procède à nouveau à une évaluation de la situation).

Elle est déclenchée lorsque certains événements peuvent faire suspecter un démarrage ou une présence de TV (sinon il n'y aurait pas stimulation).

La prolongation du délai VA peut ainsi être opérée sur un ou plusieurs des critères suivants :
- ESV de type ESV1, c'est-à-dire présence d'une détection ventriculaire non précédée d'un événement auriculaire dans un délai physiologique (typiquement 31 à 300 ms),
   et/ou
- ESV de type ESV2, c'est-à-dire présence d'un événement ventriculaire présentant un intervalle de couplage inférieur ou égal à l'intervalle de détection de TV le plus long programmé (correspondant donc à la fréquence minimale de détection de TV), avec détection d'une accélération d'origine ventriculaire (le critère d'analyse de l'accélération du rythme ventriculaire et la détermination de l'origine de cette accélération, ventriculaire ou auriculaire, sont décrits dans le EP-A-0 626 182 précité),
   et/ou
- ESV de type ESV3, c'est-à-dire détection d'un événement ventriculaire présentant un intervalle de couplage inférieur ou égal à l'intervalle de détection de TV le plus long programmé, précédé d'une ESV et avec un intervalle de couplage voisin (à typiquement ± 31 ms) de cette ESV,
   et/ou
- détection confirmée d'un rythme de TV (conformément au mode d'analyse du EP-A-0 838 235, par exemple).

Outre ce cas général, des cas particuliers peuvent se présenter, qu'il est souhaitable de prendre en compte.

Ainsi, sur une ESV, on peut envisager une stimulation auriculaire synchrone pour prévenir une conduction rétrograde. Dans ce cas, c'est l'intervalle d'échappement auriculaire IEA (c'est-à-dire le délai AA) déclenché sur cette stimulation auriculaire, qui est prolongé pour se terminer à un instant correspondant au plus tardif de :
- la fin de l'intervalle d'échappement auriculaire calculé ;
- la fin de l'intervalle maximal programmé de détection de TV, augmenté d'une marge de sécurité.

Par ailleurs, dans le cas d'un stimulateur double chambre, lorsque le stimulateur opère en mode VVI et que survient un événement ventriculaire détecté présentant un intervalle de couplage inférieur à l'intervalle de détection de TV et présentant une accélération d'origine ventriculaire (au sens exposé plus haut), pour ne pas stimuler trop tôt le ventricule c'est l'intervalle d'échappement ventriculaire qui est prolongé de manière à se terminer à un instant correspondant au plus tardif de :
- la fin de l'intervalle d'échappement ventriculaire calculé ;
- la fin de l'intervalle maximal programmé de détection de TV, augmenté d'une marge de sécurité.

## Revendications

1. Un dispositif médical implantable actif du type défibrillateur, cardiover teur et/ou stimulateur antitachycardique implantable, comprenant :
- des moyens de recueil de l'activité cardiaque ventriculaire et auriculaire,
- des moyens de thérapie antitachycardique, aptes à appliquer un choc de défibrillation, un choc de cardioversion etlou une stimulation antitachycardique,
- des moyens de stimulation antibradycardique, aptes à délivrer des impulsions de stimulation ventriculaire et/ou auriculaire à une fréquence de stimulation déterminée par le dispositif selon l'activité du patient, cette fréquence de stimulation étant au plus égale à une fréquence maximale de stimulation préprogrammée,
la cadence des impulsions délivrées étant définie d'après un intervalle d'échappement calculé incluant un délai atrio-ventriculaire et un délai ventriculo-auriculaire,
- des premiers moyens d'analyse, mis en oeuvre lorsque les moyens de recueil indiquent une fréquence d'activité cardiaque ventriculaire supérieure à une fréquence de seuil d'analyse donnée,
ces premiers moyens d'analyse étant aptes à reconnaître et discriminer la présence d'une tachycardie ventriculaire, ou d'une fibrillation ventriculaire, et à commander en conséquence les moyens de thérapie antitachycardique,
dispositif **caractérisé en ce que** ladite fréquence maximale de stimulation préprogrammée est supérieure à ladite fréquence de seuil d'analyse,
et **en ce qu'**il comprend en outre :
- des seconds moyens d'analyse du rythme cardiaque, aptes à détecter dans le rythme cardiaque une succession particulière d'événements susceptibles de révéler la présence ou l'apparition d'une tachycardie ventriculaire,
ces seconds moyens d'analyse étant aptes à prolonger la durée dudit délai ventriculo-auriculaire au moins jusqu'à la fin dudit intervalle d'échappement calculé et au moins jusqu'à la fin d'un intervalle maximal préprogrammé de détection de tachycardies ventriculaires, correspondant à ladite fréquence de seuil d'analyse, augmenté d'un délai de tolérance.

2. Le dispositif de la revendication 1, dans lequel les seconds moyens d'analyse du rythme cardiaque sont des moyens aptes à détecter la survenue d'une extrasystole ventriculaire.

3. Le dispositif de la revendication 2, dans lequel les seconds moyens d'analyse du rythme cardiaque sont des moyens aptes à détecter la survenue d'un événement ventriculaire présentant un intervalle de couplage inférieur ou égal audit intervalle maximal préprogrammé de détection de tachycardies ventriculaires, en présence d'une accélération avérée du rythme d'origine ventriculaire.

4. Le dispositif de la revendication 2, dans lequel les seconds moyens d'analyse du rythme cardiaque sont des moyens aptes à détecter la survenue d'un événement ventriculaire présentant un intervalle de couplage inférieur ou égal audit intervalle maximal préprogrammé de détection de tachycardies ventriculaires, en présence d'une extrasystole ventriculaire précédant cet événement, la durée dudit intervalle de couplage étant égale, à un facteur de tolérance donné près, à la durée séparant l'extrasystole de l'événement ventriculaire.

5. Le dispositif de la revendication 1, dans lequel les seconds moyens d'analyse du rythme cardiaque sont des moyens aptes à détecter la présence d'une rythme confirmé de tachycardie ventriculaire.

6. Le dispositif de la revendication 1, dans lequel les seconds moyens d'analyse sont aptes à prolonger la durée dudit délai ventriculo-auriculaire jusqu'au plus tardif des deux instants correspondant, d'une part, à la fin de l'intervalle d'échappement calculé et, d'autre part, à la fin de l'intervalle maximal préprogrammé de détection de tachycardies ventriculaires, augmenté d'un facteur de sécurité prédéterminé.

7. Le dispositif de la revendication 6, dans lequel ledit facteur de sécurité prédéterminé est une durée fixe préprogrammée.

8. Le dispositif de la revendication 2, dans lequel, lorsque les moyens de stimulation antibradycardique délivrent des impulsions de stimulation auriculaires synchrones d'une extrasystole ventriculaire :
- les seconds moyens d'analyse sont aptes à prolonger la durée de l'intervalle d'échappement auriculaire déclenché sur une stimulation auriculaire synchrone,
cette prolongation étant opérée jusqu'au plus tardif des deux instants correspondant, d'une part, à la fin de l'intervalle d'échappement auriculaire calculé et, d'autre part, à la fin de l'intervalle maximal préprogrammé de détection de tachycardies ventriculaires, augmenté d'un facteur de sécurité prédéterminé.

9. Le dispositif de la revendication 1, dans lequel, lorsque les moyens de stimulation antibradycardique opèrent en mode VVI :
- les seconds moyens d'analyse sont aptes à prolonger la durée de l'intervalle d'échappement ventriculaire déclenché sur un événement ventriculaire présentant un intervalle de couplage inférieur à l'intervalle maximal préprogrammé de détection de tachycardies ventriculaires, et en présence d'une accélération du rythme d'origine ventriculaire,
cette prolongation étant opérée jusqu'au plus tardif des deux instants correspondant, d'une part, à la fin de l'intervalle d'échappement ventriculaire calculé et, d'autre part, à la fin de l'intervalle maximal préprogrammé de détection de tachycardies ventriculaires, augmenté d'un facteur de sécurité prédéterminé.

## Claims

1. An active implantable medical device of the defibrillator, cardioverter and/or implantable antitachycardia pacemaker type; including:
- means for sensing ventricular and atrial cardiac activity;
- means for antitachycardia therapy able to apply a defibrillation shock, a cardioversion shock and/or an antitachycardia stimulation;
- means for antibradycardia stimulation able to deliver ventricular and/or atrial stimulation pulses at a stimulation frequency determined by the device according to patient activity, said stimulation frequency being at most equal to a maximum pre-programmed stimulation frequency, the rate of the delivered pulses being defined according to a calculated escape interval including an atrio-ventricular delay and a ventriculo-atrial delay;
- first analyzing means, which is utilised when the sensing means indicates a frequency of veritricular cardiac activity ' greater than a given threshold frequency of analysis, said first analyzing means being able to recognize and discriminate the presence of a ventricular tachycardia or a ventricular fibrillation, and to consequently command the means for antitachycardia therapy;
device **characterised in that** said maximum pre-programmed stimulation frequency is higher than said threshold frequency of analysis, an **in that** it further includes:
- second means for analyzing the cardiac rhythm able to detect in said cardiac rhythm a particular succession of events susceptible to reveal the presence or appearance of a ventricular tachycardia, said second analyzing means being able to prolong the duration of the ventriculo-atrial delay at least until the end of said calculated escape interval and at least until the end of a pre-programmed maximum interval of detection of ventricular tachycardia, corresponding to the aforementioned threshold frequency of analysis increased by a tolerance delay.

2. The device of claim 1, wherein said second means for analyzing the cardiac rhythm is a means able to detect the occurrence of a ventricular extrasystole.

3. The device of claim 2, wherein said second means for analyzing the cardiac rhythm is a means able to detect the occurrence of a ventricular event presenting a coupling interval less than or equal to the maximum pre-programmed interval of detection of ventricular tachycardia, in the presence of a proven acceleration of the rhythm having a ventricular origin.

4. The device of claim 2, wherein said second means for analyzing the cardiac rhythm is a means able to detect the occurrence of a ventricular event presenting a coupling interval less than or equal to the maximum pre-programmed interval of detection of ventricular tachycardia, in the presence of a ventricular extrasystole preceding said event, the duration of said coupling interval being equal to a duration separating the extrasystole from the ventricular event within a given tolerance factor.

5. The device of claim 1, wherein said second means for analyzing the cardiac rhythm is a means able to detect the presence of a confirmed rhythm of ventricular tachycardia.

6. The device of claim 1, wherein said second analyzing means is able to prolong the duration of said ventriculo-atrial delay until the later of the two instants corresponding, on the one hand, to the end of the calculated escape interval and, on the other hand, to the end of the pre-programmed maximum interval of detection of ventricular tachycardia, increased by a predetermined safety factor.

7. The device of claim 6, wherein said predetermined safety factor is a pre-programmed fixed duration.

8. The device of claim 2, wherein, when the antibradycardia stimulation means delivers atrial stimulation pulses synchronous to a ventricular extrasystole:
said second analyzing means is able to prolong the duration of the atrial escape interval started on a synchronous atrial stimulation, said prolongation being effected until the later of the two instants corresponding, on the one hand, to the end of the calculated atrial escape interval and, on the other hand, to the end of the pre-programmed maximum interval of detection of ventricular tachycardia, increased by a predetermined safety factor.

9. The device of claim 1, wherein, when the antibradycardia stimulation means operates in a VVI mode:
said second analyzing means is able to prolong the duration of the ventricular escape interval started on a ventricular event presenting a coupling interval less than the pre-programmed maximum interval of detection of ventricular tachycardia, and in the presence of an acceleration of the rhythm having a ventricular origin, said prolongation being effected until the later of the two instants corresponding, on the one hand, to the end of the calculated ventricular escape interval and, on the other hand, to the end of the pre-programmed maximum interval of detection of ventricular tachycardia, increased by a predetermined safety factor.

## Patentansprüche

1. Aktive implantierbare medizinische Vorrichtung vom Typ Defibrillator, Kardioverter und/oder implantierbarer antitachykardischer Herzschrittmacher, mit:
- Mitteln zur Aufnahme der Herzkammer- und Herzvorhofaktivtät,
- Mitteln zur antitachykardischen Therapie, die geeignet sind, einen Defibrillationsschock, einen Kardioversionsschock und/oder eine antitachykardische Stimulation abzugeben,
- Mitteln zur antibradykardischen Stimulation, die geeignet sind, Impulse zur Herzkammer- und/oder Herzvorhofstimulation mit einer Stimulationsfrequenz abzugeben, die von der Vorrichtung entsprechend der Aktivität des Patienten festgelegt wird, wobei diese Stimulationsfrequenz höchstens gleich einer vorprogrammierten maximalen Stimulationsfrequenz ist, wobei der Takt der abgegebenen Impulse anhand eines berechneten Ablassintervalls definiert ist, der eine atrioventrikuläre Verzögerung und eine Herzkammer-Herzvorhof-Verzögerung umfasst,
- ersten Analysemitteln, die eingesetzt werden, wenn die Aufnahmemittel eine Frequenz der Herzkammeraktivität anzeigen, die höher ist, als eine gegebene Schwellenfrequenz für die Analyse, wobei diese ersten Analysemittel geeignet sind, das Vorliegen einer ventrikulären Tachykardie oder eines Kammerflimmems zu erkennen und zu unterscheiden, und darausfolgend die Mittel zur antitachykardischen Therapie zu befehligen,
Vorrichtung, die **dadurch gekennzeichnet ist, dass** die vorprogrammierte maximale Stimulationsfrequenz höher ist als die Schwellenfrequenz für die Analyse, und dadurch, dass sie außerdem folgendes umfasst:
- zweite Mittel zur Analyse des Herzrhythmusses, die geeignet sind, im Herzrhythmus eine besondere Abfolge von Ereignissen zu erfassen, die das Vorliegen oder das Auftreten einer ventrikulären Tachykardie aufdecken können, wobei diese zweiten Analysemittel geeignet sind, die Dauer der Herzkammer-Herzvorhof-Verzögerung mindestens bis zum Ende des berechneten Ablassintervalls und mindestens bis zum Ende eines vorprogrammierten maximalen Intervalls zur Erfassung ventrikulärer Tachykardien zu verlängern, entsprechend der Schwellenfrequenz für die Analyse, erhöht um eine Toleranzverzögerung.

2. Vorrichtung nach Anspruch 1, in welcher die zweiten Mittel zur Analyse des Herzrhythmusses Mittel sind, die geeignet sind, das Auftreten einer ventrikulären Extrasystole zu erfassen.

3. Vorrichtung nach Anspruch 2, in welcher die zweiten Mittel zur Analyse des Herzrhythmusses Mittel sind, die geeignet sind, das Auftreten eines Herzkammerereignisses zu erfassen, das ein Kopplungsintervall aufweist, welches geringer oder gleich dem vorprogrammierten maximalen Intervall zur Erfassung ventrikulärer Tachykardien ist, bei Vorliegen einer bestätigten Beschleunigung des von einer Herzkammer stammenden Rhythmusses.

4. Vorrichtung nach Anspruch 2, in welcher die zweiten Mittel zur Analyse des Herzrhythmusses Mittel sind, die geeignet sind, das Auftreten eines Herzkammerereignisses zu erfassen, das ein Kopplungsintervall aufweist, welches geringer oder gleich dem vorprogrammierten maximalen Intervall zur Erfassung ventrikulärer Tachykardien ist, bei Vorliegen einer ventrikulären Extrasystole, die diesem Ereignis vorangeht, wobei die Dauer des Kopplungsintervalls, abgesehen von einem gegebenen Toleranzfaktor, gleich der Dauer ist, welche die Extrasystole von dem Herzkammerereignis trennt.

5. Vorrichtung nach Anspruch 1, in welcher die zweiten Mittel zur Analyse des Herzrhythmusses Mittel sind, die geeignet sind, das Vorliegen eines bestätigten Rhythmusses einer ventrikulären Tachykardie zu erfassen.

6. Vorrichtung nach Anspruch 1, in welcher die zweiten Analysemittel geeignet sind, die Dauer der Herzkammer-Herzvorhof-Verzögerung bis zum späteren der zwei Augenblicke zu verlängern, die einerseits dem Ende des berechneten Ablassintervalls und andererseits dem Ende des vorprogrammierten maximalen Intervalls zur Erfassung ventrikulärer Tachykardien, erhöht um einen vorbestimmten Sicherheitsfaktor, entsprechen.

7. Vorrichtung nach Anspruch 6, in welcher der vorbestimmte Sicherheitsfaktor eine feste vorprogrammierte Dauer ist.

8. Vorrichtung nach Anspruch 2, in welcher, wenn die Mittel zur antibradykardischen Stimulation zu einer ventrikulären Extrasystole synchrone Impulse zur Herzvorhofstimulation abgeben:
die zweiten Analysemittel geeignet sind, die Dauer des Herzvorhofablassintervalls zu verlängern, der bei einer synchronen Herzvorhofstimulation ausgelöst wird, wobei diese Verlängerung bis zum späteren der zwei Augenblicke durchgeführt wird, die einerseits dem Ende des berechneten Herzvorhofablassintervalls und andererseits dem Ende des vorprogrammierten maximalen Intervalls zur Erfassung ventrikulärer Tachykardien, erhöht um einen vorbestimmten Sicherheitsfaktor, entsprechen.

9. Vorrichtung nach Anspruch 1, in welcher, wenn die Mittel zur antibradykardischen Stimulation im WI-Modus arbeiten:
die zweiten Analysemittel geeignet sind, die Dauer des Herzkammerablassintervalls zu verlängern, der bei einem Herzkammerereignis ausgelöst wird, das ein Kopplungsinterval aufweist, welches geringer als das vorprogrammierte maximale Intervall zur Erfassung ventrikulärer Tachykardien ist, und bei Vorliegen einer Beschleunigung des von einer Herzkammer stammenden Rhythmusses, wobei diese Verlängerung bis zum späteren der zwei Augenblicke durchgeführt wird, die einerseits dem Ende des berechneten Herzkammerablassintervalls und andererseits dem Ende des vorprogrammierten maximalen Intervalls zur Erfassung ventrikulärer Tachykardien, erhöht um einen vorbestimmten Sicherheitsfaktor, entsprechen.
